# EUROPEAN PATENT APPLICATION

(11) **EP 3 511 050 A1**
(43) Date of publication of application: **17.07.2019**
(21) Application number: 19151195.5
(22) Date of filing: 10.01.2019
(51) Int. Cl.: A61N 1/05, A61M 25/02

(54) **LEAD ANCHOR AND SUTURE DEVICE**

(30) Priority: 11.01.2018 US 201862616236 P
(71) Applicant: Heraeus Medical Components LLC, St. Paul, MN 55127 (US)
(72) Inventor: Nelson, Randall S., Pine Springs, Minnesota 55115 (US); Zwiers, Lynn, Lino Lakes, Minnesota 55038 (US); McSherry, Paul, Woodbury, Minnesota 55125 (US)
(74) Representative: Maiwald Patent- und Rechtsanwaltsgesellschaft mbH

(57) **Abstract**

An anchor for an electrical lead or other elongate medical instrument can include an anchor body. The anchor body can define an instrument passageway. The instrument passageway can extend from a first end of the anchor body to a second end of the anchor body. The anchor body can define one or more suture holes in the anchor body. A clamp can be included in or coupled to the anchor body. The clamp can include a first clamping flange and a second clamping flange. The first clamping flange and the second clamping flange can project laterally from the clamp. The clamp can include a clamping cavity extending about the instrument passageway. The clamp can be arranged to close such that changing a distance between the first clamping flange and the second clamping flange causes a dimension of the clamping cavity to decrease and hold the instrument within the instrument passageway.

## Description

### Cross-Reference to Related Application

This Utility Patent Application claims priority to U.S. Application No. 62/616,236 filed on January 11, 2018, which is incorporated herein by reference.

### Background

Electrical leads can be utilized to transmit electrical signals to or from a medical instrument, such as a pacemaker or neurostimulator. The electrical lead can be coupled with an anchor to secure, stabilize, or fix (e.g., substantially inhibit the movement of or displacement of) the electrical lead with respect to an anchor location, such as by anchoring to bodily tissue of a patient.

### Summary

The present inventors have recognized, among other things, that a problem to be solved can include fixing (e.g., anchoring) a medical instrument (e.g., an electrical lead, a neurostimulator lead, a cardiac lead, a subdermal ventricular assist device cable, or the like) at a location without damaging or affecting the integrity of the secured medical instrument such as over a period of time associated with implantation or chronic deployment. In an example, a set screw is threaded into an anchor and the set screw directly engages with a portion of an electrical lead to provide a clamping force and help inhibit or prevent the displacement of the electrical lead with respect to the anchor. The direct application of the clamping force with the set screw can damage or affect the integrity of the electrical lead, such as by weakening or severing a wire within the electrical lead. The present subject matter can help provide a solution to this problem, such as by providing an anchor for an electrical lead or other elongate medical instrument.

The anchor can include an anchor body. The anchor body can define an instrument passageway. The instrument passageway can extend from a first end of the anchor body to a second end of the anchor body. The anchor body can define one or more suture holes in the anchor body. A suture can engage with the one or more suture holes and fix the anchor with respect to an anchor point, such as bodily tissue of a patient.

The anchor can include a clamp. The clamp can be included in or coupled to the anchor body. The clamp can include a first clamping flange. The first clamping flange can project laterally from the clamp. The clamp can include a second clamping flange. The second clamping flange can project laterally from the clamp. The clamp can include a clamping cavity. The clamping cavity can extend about the instrument passageway. The clamp can be arranged to close such that changing a distance between the first clamping flange and the second clamping flange causes a dimension of the clamping cavity to decrease and to hold the instrument within the instrument passageway.

The clamp can provide a clamping force. The clamping force can hold the electrical lead within the instrument passageway of the anchor. The clamping force can be applied about a perimeter of the medical instrument. The application of the clamping force about the perimeter of the medical instrument can distribute the clamping force that is applied to the medical instrument (e.g., decrease the pressure applied to a specific point of the medical instrument, or increase the surface area that the clamping force is applied to the medical instrument). Distribution of the clamping force can help inhibit or prevent the anchor from damaging (e.g., crushing) or affecting the long-term integrity or reliability of the medical instrument when the clamp is closed and the medical instrument is held in the instrument passageway. The anchor can include a cushion. The cushion can help improve the distribution of the clamping force, and can help further enhance the integrity or reliability of the clamped medical instrument.
Aspect 1 may include or use subject matter (such as an apparatus, a system, a device, a method, a means for performing acts, or a device readable medium including instructions that, when performed by the device, may cause the device to perform acts), such as may include or use an anchor for an electrical lead or other elongate medical instrument. The anchor can include an anchor body. The anchor body can define an instrument passageway. The instrument passageway can extend from a first end of the anchor body to a second end of the anchor body. The anchor body can define one or more suture holes in the anchor body.
   The anchor can include a clamp. The clamp can be included in or coupled to the anchor body. The clamp can include a first clamping flange. The first clamping flange can project laterally, such as from the clamp. The clamp can include a second clamping flange. The second clamping flange can project laterally, such as from the clamp. The clamp can include a clamping cavity. The clamping cavity can extend about the instrument passageway. The clamp can be arranged to close. The clamp can be arranged to close such that changing a distance between the first clamping flange and the second clamping flange causes a dimension of the clamping cavity to decrease. The clamp can be arranged to close such that the clamp holds the instrument within the instrument passageway.
Aspect 2 may include or use, or may optionally be combined with the subject matter of Aspect 1, to optionally include or use a body flange. The body flange can projecting laterally, such as from the anchor body. The first clamping flange can be embedded in the body flange. The second clamping flange can be embedded in the body flange.
Aspect 3 may include or use, or may optionally be combined with the subject matter of Aspect 2 to optionally include or use that the body flange can define a first suture hole. The body flange can define a second suture hole.
Aspect 4 may include or use, or may optionally be combined with the subject matter of one or any combination of Aspects 2 through 3 to optionally include or use a pocket. The pocket can be formed in the body flange. The pocket can include a pocket cavity. The pocket cavity can be located between the first clamping flange and the second clamping flange. The pocket cavity can be arranged to allow the clamp to close. The pocket cavity can be arranged to close the pocket.
Aspect 5 may include or use, or may optionally be combined with the subject matter of one or any combination of Aspects 2 through 4 to optionally include or use a fastener. The fastener can be arranged to engage with the clamp, such as to close the clamp. The anchor can include an access hole. The access hole can be defined in the body flange. The access hole can be arranged to allow the fastener to translate with respect to the access hole, such as to allow the fastener to engage with the clamp.
Aspect 6 may include or use, or may optionally be combined with the subject matter of Aspect 5 to optionally include or use a plug. The plug can be sized and shaped to couple with the access 5 hole. The plug can be sized and shaped to close the access hole.
Aspect 7 may include or use, or may optionally be combined with the subject matter of one or any combination of Aspects 1 through 6 to optionally include or use a cushion. The cushion can be within the clamping cavity. The cushion can be aligned with the instrument passageway. The cushion can cushion the instrument within the clamping cavity.
Aspect 8 may include or use, or may optionally be combined with the subject matter of Aspect 7 to optionally include or use an electrical lead. A portion of the electrical lead can be positioned within the cushion.
Aspect 9 may include or use, or may optionally be combined with the subject matter of one or any combination of Aspects 1 through 8 to optionally include or use an electrical lead. The closing of the clamp can provide a clamping force, such as about a perimeter of the electrical lead. The clamping force can hold the electrical lead within the instrument passageway.
Aspect 10 may include or use, or may optionally be combined with the subject matter of one or any combination of Aspects 1 through 9 to optionally include or use a circumferential suture groove. The circumferential suture groove can be about the anchor body. The circumferential suture groove can be arranged to receive a portion of a suture.
Aspect 11 may include or use, or may optionally be combined with the subject matter of Aspect 10 to optionally include or use that a portion of the suture can be engaged with a portion of the circumferential suture groove.
Aspect 12 may include or use, or may optionally be combined with the subject matter of one or any combination of Aspects 10 or 11 to optionally include or use that the circumferential suture groove is aligned with a suture hole.
Aspect 13 may include or use, or may optionally be combined with the subject matter of one or any combination of Aspects 1 through 12 to optionally include or use an anchoring flange. The anchoring flange can protrude from the anchor body. The anchoring flange can define a first anchoring hole. The first anchoring hole can be formed in the anchoring flange.
Aspect 14 may include or use, or may optionally be combined with the subject matter of one or any combination of Aspects 1 through 13 to optionally include or use an electrical lead. The electrical lead can be positioned within the instrument passageway.
Aspect 15 may include or use, or may optionally be combined with the subject matter of Aspect 14 to optionally include or use that closing the clamp can couple the electrical lead anchor with the electrical lead.
Aspect 16 may include or use, or may optionally be combined with the subject matter of one or any combination of Aspects 1 through 15 to optionally include or use a suture. The suture can be positioned within at least one of the one or more suture holes.
Aspect 17 may include or use subject matter (such as an apparatus, a system, a device, a method, a means for performing acts, or a device readable medium including instructions that, when performed by the device, may cause the device to perform acts), such as may include or use an anchor for an electrical lead or other elongate medical instrument. The anchor can include an anchor body. The anchor body can define an instrument passageway. The instrument passageway can extend from a first end of the anchor body to a second end of the anchor body. The anchor body can define one or more suture holes in the anchor body. The anchor can include a clamping means. The clamping means can be included in or coupled to the anchor body. The clamping means can be adapted to hold the instrument within the instrument passageway. The clamping means can include a laterally projecting portion, such as including a tightening means. The tightening means can be adapted to tighten the clamping means, such as to hold the instrument.
Aspect 18 may include or use subject matter (such as an apparatus, a system, a device, a method, a means for performing acts, or a device readable medium including instructions that, when performed by the device, may cause the device to perform acts), such as may include or use a method for anchoring an electrical lead or other elongate medical instrument with an anchor. The anchor can include a clamp. The clamp can have a clamping cavity. The method can include positioning an electrical lead with respect to an anchor. The method can include closing the clamp, such as by changing a distance between a first clamping flange and a second clamping flange. The first clamping flange and the second clamping flange cam project laterally from the clamp. The method can include clamping the electrical lead, such as by applying a force at a location lateral to the electrical lead. The force applied at the location lateral to the electrical lead can produce a clamping force, such as about a perimeter of the electrical lead. The clamping force can hold the electrical lead within the instrument passageway.
Aspect 19 may include or use, or may optionally be combined with the subject matter of Aspect 18, to optionally include or use that positioning the electrical lead within the anchor can include translating the electrical lead through a core passageway of a cushion.
Aspect 20 may include or use, or may optionally be combined with the subject matter of one or any combination of Aspects 18 or 19 to optionally include or use that positioning the electrical lead with respect to the anchor can include positioning a portion of the electrical lead within a first end of an instrument passageway of the anchor. The method can also include that positioning the electrical lead with respect to the anchor can include passing the electrical lead through the anchor such as by translating the electrical lead through a clamping cavity of a clamp. The electrical lead can be translated through a second end of the instrument passageway.

Each of these non-limiting examples can stand on its own, or can be combined in various permutations or combinations with one or more of the other examples.

This overview is intended to provide an overview of subject matter of the present patent application. It is not intended to provide an exclusive or exhaustive explanation of the invention. The detailed description is included to provide further information about the present patent application.

### Brief Description of the Drawings

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
**Figure 1** shows a perspective view of one example of an anchor for an electrical lead or other elongate medical instrument.
**Figure 2** shows a perspective view of one example of a clamp.
**Figure 3** shows a top view of the anchor of Figure 1.
**Figure 4** shows a cross sectional view of the anchor of Figure 3 at the line 4-4.
**Figure 5** shows a cross sectional view of the anchor of Figure 3 at the line 5-5.
**Figure 6** shows one example of a method for anchoring an electrical lead or other elongate medical instrument with an anchor, including one or more the anchor described herein.

### Detailed Description

In the following Detailed Description, reference is made to the accompanying drawings, which form a part hereof, and in which is illustrated by way of illustration specific embodiments in which one embodiments may be practiced. In this regard, directional terminology, such as "top," "bottom," "front," "back," "leading," "trailing," etc., is used with reference to the orientation of the Figure(s) being described. Because components of embodiments can be positioned in a number of different orientations, the directional terminology is used for purposes of illustration and is in no way limiting. It is to be understood that other embodiments may be utilized and structural or logical changes may be made without departing from the scope of the present embodiments. The following detailed description, therefore, is not to be taken in a limiting sense, and the scope of the present embodiments are defined by the appended claims.

It is to be understood that the features of the various exemplary embodiments described herein may be combined with each other, unless specifically noted otherwise.

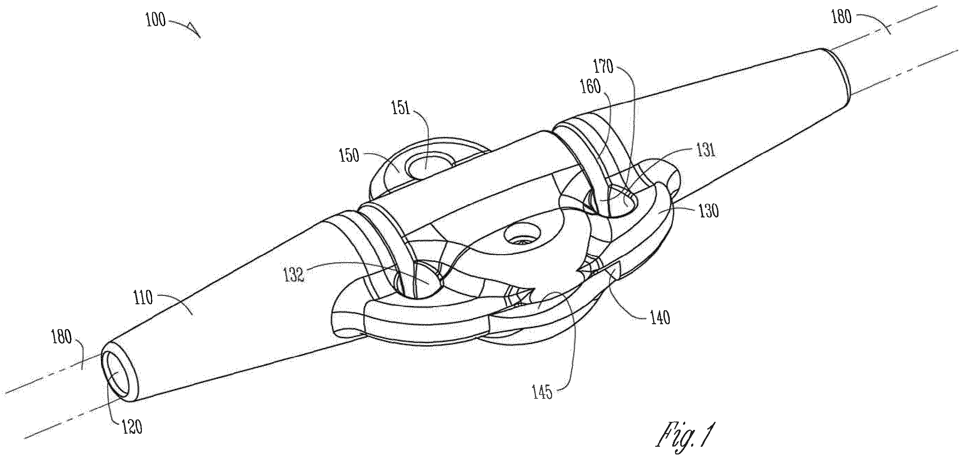

Figure 1 shows a perspective view of an example of an anchor 100 for an electrical lead 180 or other elongate medical instrument. The anchor 100 can include an anchor body 110. The anchor body 110 can include or consist of silicone or one or more other polymer materials. The anchor body 110 can define an instrument passageway 120. The instrument passageway 120 can have a circular, square, other geometric shape, or irregularly shaped cross section. The instrument passageway 120 can extend from a first end of the anchor body 110 to a second end of the anchor body 110. The instrument passageway 120 can be sized and shaped to receive a medical instrument, such as the electrical lead 180. A portion of the medical instrument can be positioned within the instrument passageway 120. An end-user (e.g., a medical professional) can position the medical instrument within the instrument passageway 120. The medical instrument can be positioned within the instrument passageway 120 by a manufacturer. The medical instrument positioned within the instrument passageway 120 can be provided to the end-user by the manufacturer. In an example, the electrical lead 180 is placed into a first end of the instrument passageway 120. The electrical lead 180 is translated through the instrument passageway and through a second end of the instrument passageway 120. The anchor 100 can be translated along the electrical lead 180 and the position of the anchor 100 with respect to the electrical lead 180 can be changed.

The instrument passageway 120 can be sized and shaped to establish a seal between the portion of the medical instrument and the instrument passageway 120, such as when the portion of the medical instrument is positioned within the instrument passageway 120. The establishment of a seal between the inserted portion of the medical instrument and the instrument passageway 120 can help inhibit or prevent debris from entering the instrument passageway 120 and the anchor 100.

The anchor 110 can include a body flange 130. The body flange 130 can project laterally from the anchor body 110. As described herein, the anchor body 110 can define one or more suture holes formed in the anchor body 110.

For example, the body flange 130 can define a first suture hole 131. The body flange 130 can define a second suture hole 132. The anchor body 110 can include an anchoring flange 150. The anchoring flange 150 can protrude from the anchor body 110. The anchoring flange 150 can define a third anchoring hole 151 formed in the anchoring flange 150.

A suture 170 can be positioned in, and engage with, the one or more suture holes, and secure or fix (e.g., substantially inhibit the movement of, or displacement of) the anchor 100 with respect to an anchor point, such as bodily tissue of a patient (not shown). The anchor 100 can include a circumferential suture groove 160. The circumferential suture groove 160 can be formed in the anchor body 110. The circumferential suture groove 160 can be about (e.g., extend around a perimeter of) the anchor body 110. As shown in Figure 1, a portion of the suture 170 can be engaged with a portion of the circumferential suture groove 170. The circumferential suture groove 160 can be adapted to receive a portion of the suture 170 (e.g., the suture 170 can be seated within the circumferential suture groove 160). The reception of the suture 170 by the circumferential suture groove 160 can help inhibit or prevent the displacement of the suture 170 with respect to the anchor 100, thereby improving the resilience of the engagement (e.g., coupling) of the suture 170 with the anchor 100.

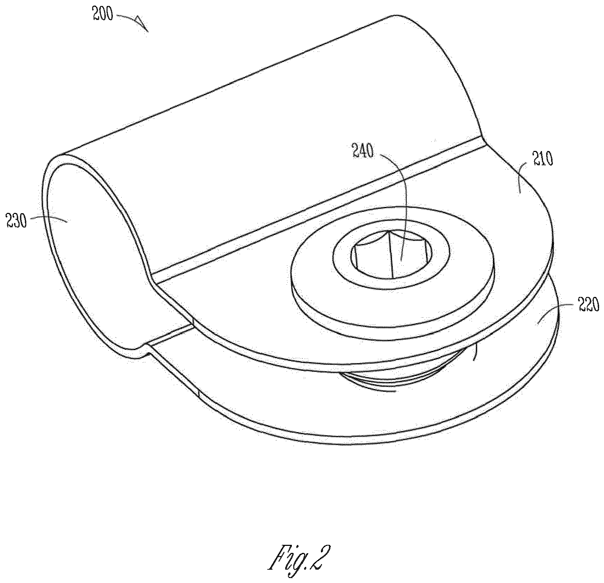

The anchor 100 can include a clamp 200 (shown in Figures 2 and 4-5). The clamp 200 can be included in or coupled to the anchor body 110. The clamp 200 can be arranged to close such as to hold a medical instrument (e.g., the electrical lead 180) within the instrument passageway 120. The clamp 200 can include a clamping cavity 230 (shown in Figures 2 and 4-5). The clamping cavity 230 can extend about the instrument passageway 120. The clamp 120 can be arranged to close. Closing the clamp 200 can hold the medical instrument within the instrument passageway 120, such as by applying a circumferential or peripheral force that can extend about the clamped portion of the instrument, such as for the entire length of the clamped portion of the instrument.

The anchor 100 can be implanted inside a patient and secure or fix a medical instrument with respect to the patient. The anchor 100 can include a pocket 140. The pocket 140 can be formed in the body flange 130. The pocket 140 can be formed in the anchoring flange 150. The pocket 140 can include a pocket cavity 145. As described herein, the pocket 140 can be arranged to allow the clamp 200 to close. The pocket 140 can be arranged to close, and thereby close the pocket cavity 145. Closing the pocket 140 can help inhibit or prevent debris (e.g., foreign material or bodily tissue) from entering the pocket cavity 145 and the anchor 100.

Figure 2 shows a perspective view of an example of a clamping means such as can include the clamp 200. The clamp can include or consist of nickel, cobalt, titanium, stainless steel, other biocompatible metals, alloys thereof (e.g., nitinol), plastic, or combinations thereof. The clamp 200 can include a first clamping flange 210. The first clamping flange 210 can project laterally 5 from the clamp 200. The clamp 200 can include a second clamping flange 220. The second clamping flange 220 can project laterally from the clamp. The first clamping flange 210 and the second clamping flange 220 can project in a substantially similar direction. The first clamping flange 210 can project parallel with respect to the second clamping flange 220. The clamp 200 can be included in, or coupled to, the anchor body 110. The clamp 200 can be embedded within or similarly integrated with the anchor 100. The first clamping flange 210 and the second clamping flange 220 can be embedded in the body flange 130. The first clamping flange 210 and the second clamping flange 220 can be embedded in the anchoring flange 150.

As described herein, the clamp 200 can be arranged to close. The closure of the clamp 200 can hold (e.g., apply a clamping force to, couple with, or the like) a medical instrument positioned within the instrument passageway 120. Additionally, the clamp 200 can include the clamping cavity 230. Further, the clamping cavity 230 can extend about (e.g., surround or form a direct interface with) the instrument passageway 120 (shown in Figure 1).

The anchor 100 can include a tightening means, such as can include the first clamping flange 210 or the second clamping flange 220. The clamping means can include the tightening means. The clamping means can include a laterally projecting portion. The laterally projecting portion can include the tightening means. The tightening means can be adapted to tighten the clamping means to hold the instrument.

Referring again to Figure 2, the clamp 200 can be arranged to close such that changing a distance between the first clamping flange 210 and the second clamping flange 220 causes a dimension of the clamping cavity 230 to decrease (e.g., bringing the first clamping flange 210 and the second clamping flange 220 together reduces the size of the clamping cavity 230). The force applied to close the clamp 200 can be applied laterally from the clamping cavity 230 (and the medical instruments positioned therein). Accordingly, the force used to close the clamp is distributed throughout the clamp 200 (as opposed to being focused at a direct interface with a set screw) and can be indirectly applied as a clamping force to the medical instrument positioned within the clamping cavity 230. Additionally, arranging the anchor 100 such that the force used to close the clamp 200 is applied laterally from the clamping cavity 230 can help reduce the profile (e.g., height) of the anchor 100.

The anchor 100 (shown in Figure 1) can be arranged such that the clamping force provided by the clamp 200 is applied about a perimeter (e.g., circumferentially) of the medical instrument (e.g., the electrical lead 180 shown in Figure 1) positioned in the instrument passageway 120. The clamping force can hold the medical instrument within the instrument passageway 120 of the anchor 100. The application of the clamping force about the perimeter of the medical instrument can distribute the clamping force that is applied to the medical instrument (e.g., decrease the pressure applied to a specific point of the medical instrument, or increase the surface area that the clamping force is applied to the medical instrument). Distribution of the clamping force can help inhibit or prevent the anchor 100 from damaging the medical instrument when the clamp 200 is closed and the medical instrument is held in the instrument passageway 120.

Referring again to Figure 2, the clamp 200 can be arranged such that decreasing a distance between the first clamping flange 210 and the second clamping flange 220 (e.g., bringing the first clamping flange 210 and the second clamping flange 220 together) causes the dimension of the clamping cavity 230 to decrease, such as by decreasing from a first dimension to a second dimension. Additionally, the clamp 200 can be biased such that the clamping cavity 230 returns to the first dimension if the force applied to the clamp 200 is removed (e.g., the first clamping flange 210 and the second clamping flange 220 are allowed to relax and are biased toward a separated configuration). The clamp 200 can be arranged such that decreasing a distance between the first clamping flange 210 and the second clamping flange 220 (e.g., bringing the first clamping flange 210 and the second clamping flange 220 together) causes the dimension of the clamping cavity 230 to increase. Increasing the dimension of the clamping cavity 230 can allow a medical instrument to enter the clamping cavity 230. The first clamping flange 210 and the second clamping flange 220 can be relaxed (e.g., the clamp 200 is closed), thereby causing the dimension of the clamping cavity 230 to decrease. The relaxing of the first clamping flange 210 and the second clamping flange 220 can hold the medical device within the clamping cavity 230.

The anchor 100 can include a fastener 240. The tightening means can include the fastener 240. The fastener 240 can be arranged to engage with the clamp 200. The engagement of the fastener 240 with the clamp 200 can close the clamp 200, such as by decreasing the distance between the first clamping flange 210 and the second clamping flange 220. The fastener 240 can be a threaded fastener. The fastener 240 and the clamp 200 can be arranged to have corresponding threads and allow the fastener 240 to engage with the clamp 200.

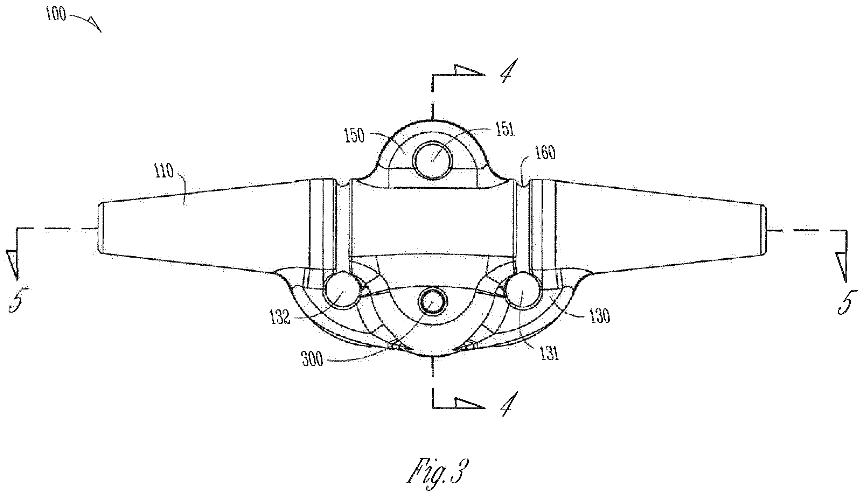

Figure 3 shows a top view of the anchor 100 of Figure 1. As described herein, the anchor 100 can include a body flange 130. The body flange 130 can include the first anchoring hole 131 and the second anchoring hole 132. The anchor 100 can include the circumferential suture groove 160. The circumferential suture groove 160 can be aligned with the one or more suture holes, such as the first anchoring hole 131 or the second anchoring hole 132. The circumferential suture groove 160 can be formed in a portion of the first anchoring hole 131 or the second anchoring hole 132.

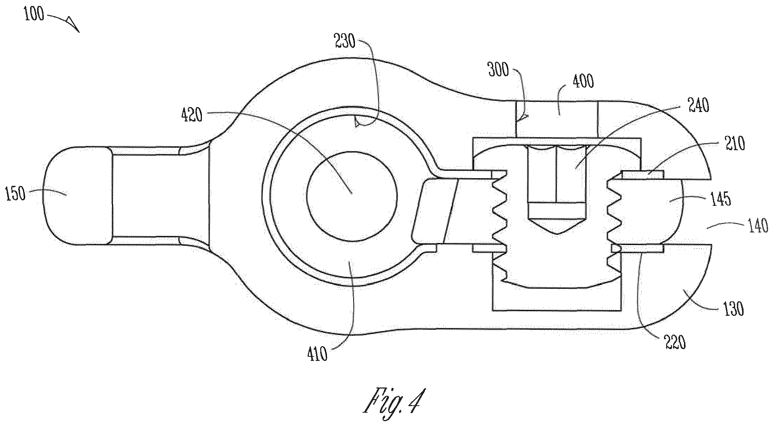

The anchor 100 can include an access hole 300. The access hole 300 can be defined in the body flange 130. The access hole 300 can be arranged to allow the fastener 240 (shown in Figures 2 and 4) to translate with respect to the access hole 300 to allow the fastener 240 to engage with the clamp 200. The fastener 240 can be embedded within the anchor 100. The access hole 300 can allow for a tool (e.g., a hex key) to engage with a portion of the fastener 240 and rotate the fastener 240 within the anchor 100, thereby closing the clamp 200. The access hole 300 can be sized and shaped to allow the fastener 240 to translate through the access hole 300 and engage with the clamp 200.

Figure 4 shows a cross sectional view of the anchor of Figure 3 at the line 4-4. The anchor 100 can include a plug 400. The plug 400 can be sized and shaped to be inserted into or to similarly couple with the access hole 300 such as to close the access hole 300. The plug 400 can be bonded (e.g., adhesively or utilizing a heat bonding process) or otherwise secured (e.g., such as with an interference fit) with the access hole 300, thereby closing the access hole 300. Closing the access hole 300 can help inhibit or prevent debris from entering the access hole 300 and the anchor 100.

As described herein, the clamp 200 can be included in, or coupled to the anchor 100. The clamp 200 can be embedded in the anchor 100. The anchor body 110 can be formed (e.g., molded) around the clamp 200. Additionally, the first clamping flange 210 and the second clamping flange 220 can be embedded in the body flange 130. Further, the anchor 100 can include the pocket 140. Still further, the pocket 140 can include the pocket cavity 145. The pocket cavity 145 can be located between the first clamping flange 210 and the second clamping flange 220. The pocket cavity 145 can be arranged to allow the clamp 200 to close. The pocket cavity 145 can provide a void to allow the first clamping flange 210 to form a direct interface with the second clamping flange 220. The closure of the clamp 200 (e.g., such as through engagement of the fastener 240 with the clamp 200) can cause a corresponding closure of the pocket 140.

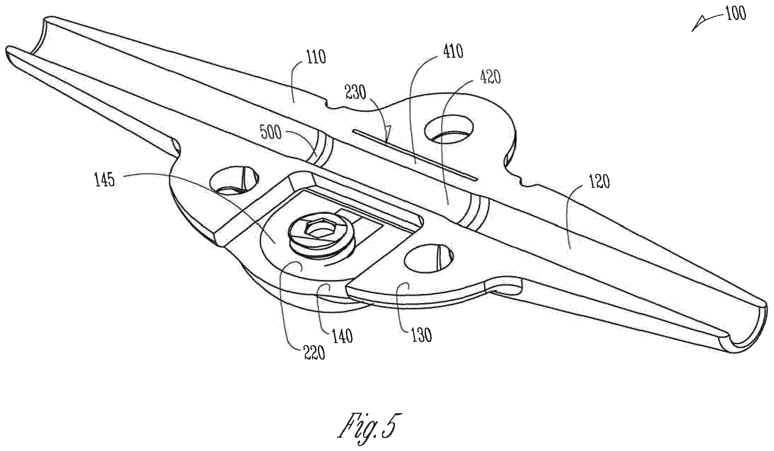

The anchor 100 can include a cushion 410. The cushion 410 can be included in, or positioned within the clamping cavity 230. The cushion 410 can define a core passageway 420. The core passageway 420 can have a dimension (e.g., diameter) within a range of 0.051 inches and 0.055 inches, however the present subject matter is not so limited. The core passageway 420 of the cushion 410 can be aligned with the instrument passageway 130 (such as shown in Figures 1 and 5).

A portion of the medical instrument (e.g., the electrical lead 180 shown in Figure 1) can be positioned within the cushion 410, such as by being positioned in the core passageway 420. The cushion 410 can improve the distribution of the clamping force provided by the clamp 200. The cushion 410 can cushion (e.g., help diminish point forces applied to, help inhibit or prevent damage to, improve the long-term reliability of, or the like) the medical instrument within the clamping cavity 230. The cushion 410 can help protect the medical instrument if an edge or an anomaly (e.g., a burr) is present within the anchor 100, such as at the interface of the clamp 200 with the anchor body 110. The cushion 410 can help protect the medical instrument if the medical instrument is strained, such as when the medical instrument is positioned within a patient and the patient moves.

Figure 5 shows a cross sectional view of the anchor of Figure 3 at the line 5-5. As described herein, the clamping cavity 230 can be aligned with (e.g., concentrically aligned) the instrument passageway 120. The clamping cavity 230 can include the cushion 410. The core passageway 420 of the cushion 410 can be aligned with the instrument passageway 120. The anchor 100 can include a transition region 500. The transition region 500 can include a tapered surface. The instrument passageway 120 can have a first dimension. The core passageway 420 can have a second dimension. The second dimension can be specified to correspond to the dimension of a medical instrument. The first dimension can be larger than the second dimension, such as to facilitate the reception of the medical device by the anchor 100. The transition region 500 can help provide a smooth transition from the first dimension to the second dimension.

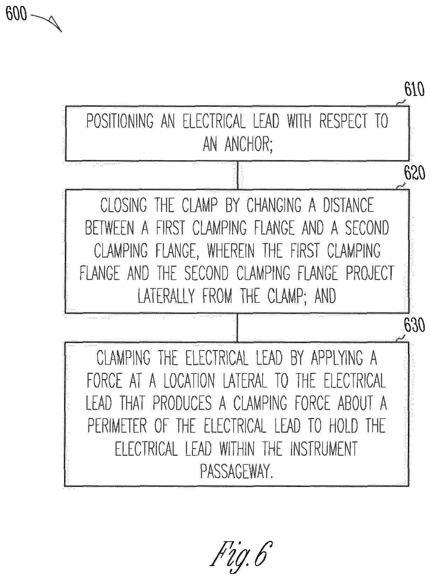

Figure 6 shows an example of a method 600 for anchoring an electrical lead or other elongate medical instrument with an anchor, including one or more the anchor described herein. In describing the method 600, reference is made to one or more components, features, functions and steps previously described herein. Where convenient, reference is made to the components, features, steps and the like with reference numerals. The reference numerals provided are exemplary and are not exclusive. For instance, components, features, functions, steps and the like described in the method 600 include, but are not limited to, the corresponding numbered elements provided herein and other corresponding elements described herein (both numbered and unnumbered) as well as their equivalents.

At 610, the method 600 can include positioning an electrical lead with respect to an anchor. The anchor can include a clamp having a clamping cavity. Positioning the electrical lead within the anchor can include translating the electrical lead through a core passageway of a cushion. Positioning the electrical lead with respect to the anchor can include positioning a portion of the electrical lead within a first end of an instrument passageway of the anchor. Positioning the electrical lead with respect to the anchor can include passing the electrical lead through the anchor such that the electrical lead is translated through a clamping cavity of a clamp and is translated through a second end of the instrument passageway.

At 620, the method 600 can include closing the clamp, such as by changing a distance between a first clamping flange and a second clamping flange. The first clamping flange and the second clamping flange can project laterally from the clamp. At 630, the method 600 can include clamping the electrical lead, such as by applying a force at a location lateral to the electrical lead (e.g., applying a force to the first clamping flange 210 and the second clamping flange 220 of Figures 2 and 4). The force can produce a clamping force about a perimeter of the electrical lead to hold the electrical lead within the instrument passageway.

### Various Notes

The above description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In the event of inconsistent usages between this document and any documents so incorporated by reference, the usage in this document controls.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

Geometric terms, such as "parallel", "perpendicular", "round", or "square", are not intended to require absolute mathematical precision, unless the context indicates otherwise. Instead, such geometric terms allow for variations due to manufacturing or equivalent functions. For example, if an element is described as "round" or "generally round," a component that is not precisely circular (e.g., one that is slightly oblong or is a many-sided polygon) is still encompassed by this description.

Method examples described herein can be machine or computer-implemented at least in part. Some examples can include a computer-readable medium or machine-readable medium encoded with instructions operable to configure an electronic device to perform methods as described in the above examples. An implementation of such methods can include code, such as microcode, assembly language code, a higher-level language code, or the like. Such code can include computer readable instructions for performing various methods. The code may form portions of computer program products. Further, in an example, the code can be tangibly stored on one or more volatile, non-transitory, or nonvolatile tangible computer-readable media, such as during execution or at other times. Examples of these tangible computer-readable media can include, but are not limited to, hard disks, removable magnetic disks, removable optical disks (e.g., compact disks and digital video disks), magnetic cassettes, memory cards or sticks, random access memories (RAMs), read only memories (ROMs), and the like.

The above description is intended to be illustrative, and not restrictive. For example, the above-described examples (or one or more aspects thereof) may be used in combination with each other. Other embodiments can be used, such as by one of ordinary skill in the art upon reviewing the above description. The Abstract is provided to comply with 37 C.F.R. §1.72(b), to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description as examples or embodiments, with each claim standing on its own as a separate embodiment, and it is contemplated that such embodiments can be combined with each other in various combinations or permutations. The scope of the invention should be determined with reference to the appended claims, along with the full scope of equivalents to which such claims are entitled.

Although specific embodiments have been illustrated and described herein, it will be appreciated by those of ordinary skill in the art that a variety of alternate and/or equivalent implementations may be substituted for the specific embodiments illustrated and described without departing from the scope of the present embodiments. This application is intended to cover any adaptations or variations of the specific embodiments discussed herein. Therefore, it is intended that these embodiments be limited only by the claims and the equivalents thereof.

## Claims

1. An anchor for an electrical lead or other elongate medical instrument, the anchor comprising:
an anchor body, defining:
an instrument passageway that extends from a first end of the anchor body to a second end of the anchor body,
one or more suture holes in the anchor body;
a clamp included in or coupled to the anchor body, the clamp including:
a first clamping flange projecting laterally,
a second clamping flange projecting laterally,
a clamping cavity extending about the instrument passageway; and
wherein the clamp is arranged to close such that changing a distance between the first clamping flange and the second clamping flange causes a dimension of the clamping cavity to decrease and to hold the instrument within the instrument passageway.

2. The anchor of claim 1, further comprising a body flange projecting laterally from the anchor body, wherein the first clamping flange and the second clamping flange are embedded in the body flange.

3. The anchor of claim 2, wherein the body flange projecting laterally from the anchor body defines a first suture hole and a second suture hole.

4. The anchor of claim 2, further comprising a pocket formed in the body flange, wherein the pocket includes a pocket cavity located between the first clamping flange and the second clamping flange, wherein the pocket cavity is arranged to allow the clamp to close and to close the pocket.

5. The anchor of claim 2, further comprising:
a fastener arranged to engage with the clamp to close the clamp; and
an access hole, defined in the body flange, and arranged to allow the fastener to translate with respect to the access hole to allow the fastener to engage with the clamp.

6. The anchor of claim 5, further comprising a plug sized and shaped to couple with the access hole to close the access hole.

7. The anchor of claim 1, further comprising a cushion within the clamping cavity and aligned with the instrument passageway to cushion the instrument within the clamping cavity.

8. The anchor of claim 7, further comprising an electrical lead, wherein a portion of the electrical lead is positioned within the cushion.

9. The anchor of claim 1, further comprising an electrical lead, wherein the closing of the clamp provides a clamping force about a perimeter of the electrical lead to hold the electrical lead within the instrument passageway.

10. The anchor of claim 1, further comprising a circumferential suture groove about the anchor body to receive a portion of a suture.

11. The anchor of claim 10, further comprising a suture, wherein a portion of the suture is engaged with a portion of the circumferential suture groove.

12. The anchor of claim 10, wherein the circumferential suture groove is aligned with a suture hole.

13. The anchor of claim 1, further comprising an anchoring flange protruding from the anchor body, wherein the anchoring flange defines a first anchoring hole formed in the anchoring flange.

14. The anchor of claim 1, further comprising an electrical lead positioned within the instrument passageway.

15. The anchor of claim 14, wherein closing the clamp couples the electrical lead anchor with the electrical lead.

16. The anchor of claim 1, further comprising a suture positioned within at least one of the one or more suture holes in the anchor body.

17. An anchor for an electrical lead or other elongate medical instrument, the anchor comprising:
an anchor body, defining:
an instrument passageway that extends from a first end of
the anchor body to a second end of the anchor body,
one or more suture holes in the anchor body; and
a clamping means included in or coupled to the anchor body,
wherein the clamping means is adapted to hold the instrument within the instrument passageway and includes a laterally projecting portion including a tightening means adapted to tighten the clamping means to hold the instrument.

18. A method for anchoring an electrical lead or other elongate medical instrument with an anchor that includes a clamp having a clamping cavity, the method comprising:
positioning an electrical lead with respect to an anchor;
closing the clamp by changing a distance between a first clamping flange and a second clamping flange, wherein the first clamping flange and the second clamping flange project laterally from the clamp; and
clamping the electrical lead by applying a force at a location lateral to the electrical lead that produces a clamping force about a perimeter of the electrical lead to hold the electrical lead within the instrument passageway.

19. The method of claim 18, wherein positioning the electrical lead within the anchor includes translating the electrical lead through a core passageway of a cushion.

20. The method of claim 18, wherein positioning the electrical lead with respect to the anchor includes:
positioning a portion of the electrical lead within a first end of an instrument passageway of the anchor; and
passing the electrical lead through the anchor such that the electrical lead is translated through a clamping cavity of a clamp and is translated through a second end of the instrument passageway.
